# EUROPEAN PATENT SPECIFICATION

(11) **EP 1 103 550 B1**
(45) Date of publication and mention of the grant of the patent: **17.01.2007**
(21) Application number: 00124585.1
(22) Date of filing: 10.11.2000
(51) Int. Cl.: C07D 311/62

(54) **Process for concentrating catechin solutions using membranes**
Verfahren zum Konzentrieren von Catechinlösungen mittels Membranen
Procédé de concentration par membranes de solutions de catéchines

(30) Priority: 16.11.1999 EP 99122753
(43) Date of publication of application: 30.05.2001
(73) Proprietor: DSM IP Assets B.V., 6411 TE Heerlen (NL)
(72) Inventor: Bonrath, Werner, 79115 Freiburg (DE); Burdick, David Carl, 4102 Binningen (CH); Schirg, Peter, 79410 Badenweiler (DE); Thum, Andreas, 89073 Ulm (DE)
(74) Representative: Schwander, Kuno

(56) References cited:
- WO-A-99/08693
- US-A- 5 879 733
- DATABASE WPI Week 199491 Derwent Publications Ltd., London, GB; AN 1994-329939 XP002158371 & JP 06 256201 A (TAIYO KAGAKU K. K.), 13 September 1994 (1994-09-13)
- DATABASE WPI Week 199421 Derwent Publications Ltd., London, GB; AN 1994-173736 XP002158372 & JP 06 116258 A (NIKKEN FOOD K. K.), 26 April 1994 (1994-04-26)
- DATABASE WPI Week 199035 Derwent Publications Ltd., London, GB; AN 1990-264451 XP002158373 & JP 02 184626 A (ITOEN K. K.), 19 July 1990 (1990-07-19)
- DATABASE WPI Week 199003 Derwent Publications Ltd., London, GB; AN 1990-019288 XP002158374 & JP 01 299224 A (ITOEN K. K.), 4 January 1928 (1928-01-04)
- DATABASE WPI Week 198711 Derwent Publications Ltd., London, GB; AN 1987-076886 XP002158375 & JP 62 030711 A (FURUKAWA NETSUGAKE), 9 February 1987 (1987-02-09)

## Description

The present invention relates to a process for concentrating aqueous epigallocatechin gallate (hereinafter abbreviated to EGCG) solutions.

In particular the invention relates to a process for concentrating aqueous EGCG solutions using selective nanofiltration or reversed osmosis membranes (herein abbreviated to membrane).

US Patent 5,879,733 describes a process for the preparation of green tea extract having improved clarity and color. These extracts are obtained by treating aqueous green tea extract with an amount of a food grade cation exchange resin effective to remove metal cations present in the extract. The extract treated with a cation exchange resin is then contacted with a nanofiltration membrane to remove the higher molecular weight materials such as e.g. pectins, proteins, chlorophyll and oxidation products. The nanofiltration membranes described in US 5,879,733 are made from polymers having a molecular weight cut off from about 700 to about 5000 Daltons (corresponding to pore sizes in the range of from about 17 to about 40 Angstroms). Polymers such as cellulose acetates, polysulfones, polyvinylidenefluorides are used for making these nanofiltration membranes. Especially disclosed is the use of an OSMO SP-12 nanofiltration membrane (made by Osmonics, Inc. of Minnetonka, Minn.) The cellulose acetate polymer from which the membrane is made has a molecular weight cut off of 1000 Daltons (corresponding to a pore size of about 20 Angstroms). By use of the membrane the tea catechins which have a molecular size smaller than the pore diameter of the membrane pass the membrane together with water, while the higher molecular weight materials are rejected by the membrane.

US 5,879,733 does not disclose any membrane which is suitable to concentrate aqueous EGCG solutions. Thus, there is still a need to find a membrane having a high retention rate of EGCG and being able to maintain an acceptable flow rate of the permeate stream.

The present invention relates to a multiple step process for concentrating aqueous epigallocatechin gallate (EGCG) solutions, which process can be performed at least partially batchwise or continuously, and comprises:
a) feeding an aqueous EGCG solution having a dissolved solids content of about 0.03 wt% to about 10 wt% to at least one membrane module under a pressure of about 5 bar to about 100 bar to provide a permeate purge and a retentate return wherein the concentration of EGCG is increased in the retentate return relative to the concentration in the feed solution and whereby the membrane is a DESAL-5 membrane;
b) collecting said permeate purge;
c) collecting said retentate return;
d) optionally recycling the retentate return back to the membrane;
e) optionally concentrating the retentate return of steps c) and/or d);
f) optionally concentrating the permeate purge of step b).

The requirements on the membrane used are: a retention coefficient for EGCG over 90%, a permeate flow rate over 5 l/m² h, a retention coefficient for the optionally added organic solvent and for any impurities being present of less than 50%.

Membranes are generally characterized in a defined solvent-membrane-system by the retention coefficient R and the permeate flow rate Jᵥ.

With regard to the concentration of an aqueous EGCG solution the retention coefficient R is defined as R = 100 x (1-Cₚ/Cₖ), wherein Cₚ is the concentration of EGCG in the permeate and Cₖ is the concentration of EGCG in the retentate.

The permeate flow is a function of the osmotic pressure, which is caused by all dissolved molecules (e.g. EGCG and organic solvent) in the concentrate.

The permeate flow Jᵥ is defined as Jᵥ = A x (ΔP-ΔΠ), wherein
A is a membrane constant in l/m² h bar,
ΔP is the transmembrane difference of the pressure,
ΔΠ is the transmembrane difference of the osmotic pressure.

The necessary requirements on the membrane are fulfilled by the membrane DESAL-5 which is commercially available from OSMONICS / DESAL, Vista, California. The DESAL-5 membrane permits concentration of EGCG in a range up to 200 fold.

A preferred pressure range for the membrane is from about 10 bar to about 100 bar, more preferably from about 20 bar to about 35 bar.

The process of the invention may be operated at any suitable and desired temperature selected from about 10° C to about 60° C.

The term "aqueous EGCG solution" refers in this context to a solution of EGCG in a mixture of water and optionally an organic solvent. The solvent or the solvent mixture preferably comprises about 70 vol% to about 100 vol%, preferably about 90 vol% of water and about 0 vol% to about 35 vol%, preferably about 10 vol% of an organic solvent. The organic solvent is preferably methanol, ethanol, isopropanol or acetone. The initial concentration of EGCG in the solution is about 0.05 wt% to about to about 10wt%, preferably about 0.05 wt% to about 2 wt%.

Starting material is a green tea extract which can be prepared by methods known in the art e.g. by extracting green tea leaves. Tea extract powders are also commercially available e.g. from Highyin Biological Products Co., Guiyang, China.

EGCG is separated from tea polyphenols present in the extract by chromatography e.g. as described in the European Patent Application 99116032.6 referring to a process for producing EGCG which comprises the steps of
a) providing a green tea extract;
b) subjecting the green tea extract to a chromatography on a macroporous polar resin at a temperature in the range of about 30° C to about 80° C;
c) eluting EGCG from the macroporous polar resin with a polar elution solvent at a temperature in the range of about 30° C to about 80° C and at a pressure in the range of about 0.1 bar to about 50 bar;
d) optionally concentrating the eluate of step c);
e) optionally regenerating the macroporous polar resin by desorbing the remaining catechins; and
f) optionally concentrating the desorbed catechins of step e).

A possible embodiment of the above chromatography process is described in Example 1.

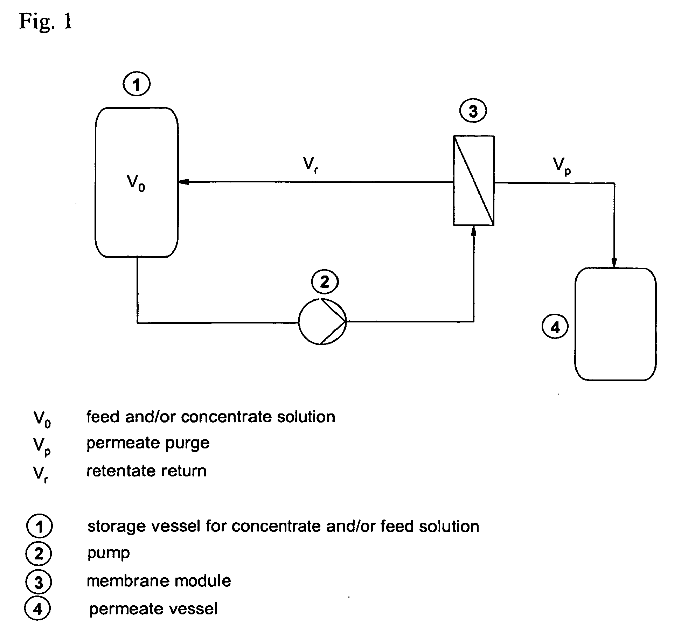

The process of the present invention will now be set forth in greater detail with reference to Figure 1 showing schematically a single stage membrane nanofiltration device.

The device comprises a feed solution vessel (1) connected to a membrane module (3) by a feed conduit through a high pressure pump (2). Module (3) comprises a spiral wound type or flat sheet type module containing the filtration membrane. The permeate purge conduit Vₚ is connected to permeate vessel (4). The return conduit Vᵣ is used for recycle of the retentate return to vessel (1). Preferred is a spiral wound module.

In operation, aqueous feed solution containing EGCG is passed to module (3) under a pressure of about 5 to about 100 bar by pump (2). Exiting through Vp is the permeate purge containing water and optionally an organic solvent. Exiting through conduit Vᵣ is the retentate return containing EGCG, which does not pass through the membrane.

For the process depicted in Fig. 1 the retentate return may be sent to the membrane process again, in one or more cycles, in either a batch or a multiple step process (additional concentration of the permeate purge) or as a continuous process.

The following examples explain the invention in more detail.

### Example 1 Preparation of the starting material.

A chromatography column was filled with 2.2 kg of Amberlite® XAD-7 resin. 50 g green tea extract from Highyin Biological Products Co., Guiyang, China were dissolved in 50 ml deionized water and the obtained solution was adsorbed onto the top of the column at 60° C and subsequently eluted at 60° C under an argon atmosphere by an eluent flow of 0.1 l/min. The eluent was a mixture of water/isopropanol (ratio 9:1 by volume). The eluent was degassed and kept under an argon atmosphere prior to use. Three main fractions were obtained containing
a) 10.0 g EGCG in 10.3 1 solution (0.97 g EGCG/l)
b) 5.2 g EGCG in 10.4 l solution (0.50 g EGCG/1)
c) 1.3 g EGCG in 5.6 l solution (0.23 g EGCG/1).

The amount of EGCG was determined by HPLC analysis. Solvents containing specific amounts of EGCG were used as external standards to quantitatively determine the amount of EGCG by HPLC in the above fractions. The fractions were combined, resulting in a feed solution containing 0.63 g EGCG/1 water/isopropanol 9/1.

### Example 2a: Concentrating the EGCG solution in a spiral wound membrane module

Storage vessel (1) was filled with the feed solution of Example 1. The starting volume of feed solution was 61. The rest of the feed solution was added to the membrane module in 2 1-portions when 2 1 permeate were obtained The solution was passed at ambient temperature through the membrane module (dead volume about 2 1) having a spiral wound membrane element DESAL DL 2540 F containing a DESAL 5 DL membrane. The permeate flow rate was initially 12.2 1/m² h and at the end 6.8 l/m² h. The pressure across the membrane was kept constant at 31 bar. The membrane surface was 2.5 m². The retentate return was returned to vessel (1). 26.3 1 feed solution containing 0.63 g EGCG/l were concentrated to 2.061. The concentrate was found to have minimum 15.6 g EGCG; minimum yield 95%. No EGCG could be detected by HPLC in the permit purge (the limit of detection was about 0.001 mg/ml). The retention coefficient was therefore about 99.99. The concentration factor was about 13.

### Example 2b: Concentrating the EGCG solution in a flat sheet membrane module (optionally)

For practical reasons a flat sheet membrane module with about 0.1 l dead volume was used for further concentration of the concentrate obtained in Example 2a.

Storage vessel (1) was filled with the feed solution of Example 2a. The starting volume of feed solution was 0.61. The rest of the feed solution was added continuously under nitrogen purge until 14.7 g dissolved EGCG were added. The solution was passed at ambient temperature through the membrane module having a flat sheet membrane element DESAL DL 5. The permeate flow rate was initially 16 l/m² h and at the end 3.8 1/m² h. The pressure across the membrane was kept constant at 43 bar. The membrane surface was 0.0028 m². The retentate return was returned to vessel (1) and concentrated to an end volume of 126 ml. The concentrate was found to have minimum 14.2 g EGCG; minimum yield 97%. Traces of EGCG were detected by HPLC in the permeate purge (the limit of quantification was about 0.01 mg/ml). The retention coefficient was therefore about 99.99. The concentration factor was about 16.

### Example 3

A feed solution containing 0.98 g EGCG/l water/isopropanol 9/1 was concentrated in a flat sheet membrane module at 35 bar and ambient temperature as described in Example 2b. The retention coefficient was 99.8.

### Example 4:

A feed solution containing 0.98 g EGCG/l water/isopropanol 9/1 (v/v) was concentrated in a flat sheet membrane module at 35 bar and ambient temperature using the following commercially available membranes. The NITTO membranes are available from Nitto Denko, Japan. The membrane surface was 0.0028 m². The following table shows the results, also including the result of Example 3.

| Membrane | permeate flow rate (beginning) 1/m² h | retention (beginning) % | permeate flow rate (end) 1/m² h | retention (end) % |
|---|---|---|---|---|
| NITTO 7250 NF | 19 | 93.8 | 8 (after 6.5 h) | 96.9 |
| DESAL 5 DL NF | 22 | >99.1 | 10 (after 6.5 h) | > 99.8 |
| NITTO 7450 NF | 32 | 95.0 | 10 (after 7 h) | 91.1 |
| DESAL YK NF | 23 | 88.0 | 8 (after 7 h) | 86.3 |

The above table clearly shows that the nanofiltration membrane DESAL 5 DL NF achieves an unexpectedly high retention of EGCG, while maintaining an acceptable flow rate of the feed solution to be filtered.

## Claims

1. A process for concentrating an aqueous epigallocatechin gallate (EGCG) solution which process comprises:
a) feeding an aqueous EGCG solution having a dissolved solids content of about 0.03 wt% to about 10 wt% to at least one membrane module under a pressure of about 5 bar to about 100 bar to provide a permeate purge and a retentate return wherein the concentration of EGCG is increased in the retentate return relative to the concentration in the feed solution and whereby the membrane is a DESAL-5 membrane;
b) collecting said permeate purge;
c) collecting said retentate return;
d) optionally recycling the retentate return back to the membrane;
e) optionally concentrating the retentate return of steps c) and/or d);
f) optionally concentrating the permeate purge of step b).

2. A process according to claim 1, wherein the membrane module is a spiral wound membrane element

3. A process according to claim 1 or 2, wherein the pressure range for the membrane is from about 10 bar to about 100 bar.

4. A process according to claim 1 or 2, wherein the pressure range for the membrane is from about 20 bar to about 35 bar.

5. A process according to any one of claims 1 to 4, wherein the process is operated at a temperature from about 10° C to about 60° C.

6. A process according to any one of claims 1 to 5, wherein the aqueous EGCG solution is a solution of EGCG in a mixture of water and optionally an organic solvent.

7. A process according to claim 6, wherein the mixture comprises about 70 vol% to about 100 vol%, preferably about 90 vol% of water and about 0 vol% to about 35 vol%, preferably about 10 vol% of an organic solvent.

8. A process according to claim 6 or 7, wherein the organic solvent is methanol, ethanol, isopropanol or acetone.

## Patentansprüche

1. Verfahren zum Konzentrieren einer wässrigen Epigallocatechingallat- (EGCG) -lösung, wobei das Verfahren umfasst:
a) Einspeisen einer wässrigen EGCG-Lösung mit einem Gehalt an gelösten Feststoffen von etwa 0,03 Gew.-% bis etwa 10 Gew.-% in mindestens ein Membranmodul unter einem Druck von etwa 5 bar bis etwa 100 bar, um ein abgeführtes Permeat und ein zurückgeführtes Retentat bereitzustellen, wobei die Konzentration von EGCG in dem zurückgeführten Retentat im Verhältnis zu der Konzentration in der Einspeiselösung erhöht ist, und wobei die Membran eine DESAL-5-Membran ist;
b) Sammeln des abgeführten Permeats;
c) Sammeln des zurückgeführten Retentats;
d) gegebenenfalls Rezirkulieren des zurückgeführten Retentats zurück zu der Membran;
e) gegebenenfalls Konzentrieren des zurückgeführten Retentats der Schritte c) und/oder d);
f) gegebenenfalls Konzentrieren des abgeführten Permeats von Schritt b).

2. Verfahren nach Anspruch 1, wobei das Membranmodul ein spiralförmiges Membranelement ist.

3. Verfahren nach Anspruch 1 oder 2, wobei der Druckbereich für die Membran bei etwa 10 bar bis etwa 100 bar liegt.

4. Verfahren nach Anspruch 1 oder 2, wobei der Druckbereich für die Membran bei etwa 20 bar bis etwa 35 bar liegt.

5. Verfahren nach einem der Ansprüche 1 bis 4, wobei das Verfahren bei einer Temperatur von etwa 10 °C bis etwa 60 °C ausgeführt wird.

6. Verfahren nach einem der Ansprüche 1 bis 5, wobei die wässrige EGCG-Lösung eine Lösung von EGCG in einer Mischung von Wasser und gegebenenfalls einem organischen Lösemittel ist.

7. Verfahren nach Anspruch 6, wobei die Mischung etwa 70 Vol.-% bis etwa 100 Vol.-%, vorzugsweise etwa 90 Vol.-%, Wasser und etwa 0 Vol.-% bis etwa 35 Vol.-%, vorzugsweise etwa 10 Vol.-%, eines organischen Lösemittels umfasst.

8. Verfahren nach Anspruch 6 oder 7, wobei das organische Lösemittel Methanol, Ethanol, Isopropanol oder Aceton ist.

## Revendications

1. Procédé de concentration d'une solution aqueuse de gallate d'épigallocatéchine (EGCG), lequel procédé comprend :
a) l'alimentation avec une solution aqueuse d'EGCG possédant une teneur en matières solides dissoutes d'environ 0,03 % en poids à environ 10 % en poids d'au moins un module de membrane sous une pression d'environ 5 bars à environ 100 bars pour fournir une purge de perméat et un retour de rétentat, la concentration d'EGCG étant augmentée dans le retour de rétentat par rapport à la concentration dans la solution d'alimentation et la membrane étant une membrane DESAL-5 ;
b) la collecte de ladite purge de perméat ;
c) la collecte dudit retour de rétentat ;
d) le recyclage facultatif du retour de rétentat vers la membrane ;
e) la concentration facultative du retour de rétentat des étapes c) et/ou d) ;
f) la concentration facultative de la purge de perméat de l'étape b).

2. Procédé selon la revendication 1, dans lequel le module de membrane est un élément de membrane enroulé en spirale.

3. Procédé selon la revendication 1 ou 2, dans lequel la gamme de pression pour la membrane est d'environ 10 bars à environ 100 bars.

4. Procédé selon la revendication 1 ou 2, dans lequel la gamme de pression pour la membrane est d'environ 20 bars à environ 35 bars.

5. Procédé selon l'une quelconque des revendications 1 à 4, le procédé étant mis en oeuvre à une température d'environ 10°C à environ 60°C.

6. Procédé selon l'une quelconque des revendications 1 à 5, dans lequel la solution aqueuse d'EGCG est une solution d'EGCG dans un mélange d'eau et éventuellement d'un solvant organique.

7. Procédé selon la revendication 6, dans lequel le mélange comprend environ 70 % en volume à environ 100 % en volume, de préférence environ 90 % en volume, d'eau et environ 0 % en volume à environ 35 % en volume, de préférence environ 10 % en volume, d'un solvant organique.

8. Procédé selon la revendication 6 ou 7, dans lequel le solvant organique est le méthanol, l'éthanol, l'isopropanol ou l'acétone.
